# EUROPÄISCHE PATENTSCHRIFT

(11) **EP 2 528 684 B1**
(45) Veröffentlichungstag und Bekanntmachung des Hinweises auf die Patenterteilung: **27.06.2018**
(21) Anmeldenummer: 11701811.9
(22) Anmeldetag: 26.01.2011
(51) Int. Cl.: B01J 29/40, B01J 35/10, C07C 2/12, C07C 1/20, C10G 3/00

(54) **VERBESSERTER KATALYSATOR AUF ZEOLITHBASIS ZUR HERSTELLUNG VON OLEFINEN UND ZUR OLIGOMERISIERUNG VON OLEFINEN**
IMPROVED CATALYST BASED ON ZEOLITE FOR PRODUCING OLEFINS AND FOR OLIGOMERIZING OLEFINS
CATALYSEUR AMÉLIORÉ À BASE DE ZÉOLITHE, UTILISÉ POUR LA PRODUCTION D'OLÉFINES ET POUR L'OLIGOMÉRISATION D'OLÉFINES

(30) Priorität: 26.01.2010 DE 102010005704
(43) Veröffentlichungstag der Anmeldung: 05.12.2012
(73) Patentinhaber: Süd-Chemie IP GmbH & Co. KG, 81925 München (DE)
(72) Erfinder: BURGFELS, Götz, 83043 Bad Aibling (DE); KLINGELHÖFER, Stefan, 83022 Rosenheim (DE); ONG, Lay Hwa, 1058 XR Amsterdam (NL); OLINDO, Roberta, 60388 Frankfurt/Main (DE); LERCHER, Johannes A., 85521 Ottobrunn (DE); SCHMIDT, Friedrich, 83024 Rosenheim (DE)
(74) Vertreter: Kuba, Stefan
(86) Internationale Anmeldenummer: PCT/EP2011/051023
(87) Internationale Veröffentlichungsnummer: WO 2011/092177

(56) Entgegenhaltungen:
- EP-A2- 0 369 364
- WO-A1-02/094436
- WO-A1-2009/071654
- BJORGEN M ET AL: "Methanol to gasoline over zeolite H-ZSM-5: Improved catalyst performance by treatment with NaOH", APPLIED CATALYSIS A: GENERAL, ELSEVIER SCIENCE, AMSTERDAM, NL, Bd. 345, Nr. 1, 31. Juli 2008 (2008-07-31) , Seiten 43-50, XP022765289, ISSN: 0926-860X, DOI: DOI:10.1016/J.APCATA.2008.04.020 [gefunden am 2008-04-24]
- HIROSHI YAMAZAKI ET AL: "Evidence for a Carbene-like Intermediate during the Reaction of Methoxy Species with Light Alkenes on H-ZSM-5", ANGEWANDTE CHEMIE. INTERNATIONAL EDITION, VCH VERLAG, WEINHEIM, DE, Bd. 50, Nr. 8, 18. Februar 2011 (2011-02-18), Seiten 1853-1856, XP008135494, ISSN: 0570-0833, DOI: DOI:10.1002/ANIE.201007178 [gefunden am 2011-01-18]

## Beschreibung

Die vorliegende Erfindung betrifft neue Katalysatoren auf Zeolithbasis, sowie deren Verwendung in einem Verfahren zur Herstellung von niederen Olefinen aus Oxygenaten und in einem Verfahren zur Oligomerisierung von Olefinen.

Die Umwandlung von Oxygenaten, insbesondere von Methanol oder Dimethylether, in niedere Olefine, insbesondere Propylen, ist seit langer Zeit bekannt. Dabei werden häufig kristalline Alumosilicate als Katalysatoren eingesetzt. Die Herstellung von Propylen ist von großem wirtschaftlichem Interesse, da Propylen einen wichtigen Rohstoff für die Gewinnung von Polypropylen darstellt, wobei das aus Methanol erhaltene Propylen dem durch thermische Spaltung von Kohlenwasserstoffen erhaltenen Propylen vorzuziehen ist, da es praktisch frei von Schwefelverbindungen ist.

Ein derartiges Verfahren ist aus der US 4,058,576 bekannt. Im Einzelnen wird bei diesem Verfahren Methanol in einer ersten Stufe an einem sauren Katalysator, wie gamma-Aluminiumoxid, in einer exothermen Kondensationsreaktion mindestens teilweise in Dimethylether umgewandelt. Auf diese Weise kann ein Teil der Reaktionswärme der in der zweiten Stufe erfolgenden Umsetzung des Methanols zu niederen Olefinen abgebaut werden, da die bei der exothermen Umsetzung erzeugte Wärme bei Verwendung von Dimethylether als Ausgangsmaterial geringer ist als bei Verwendung von Methanol. In der zweiten Stufe erfolgt die Umsetzung über einem kristallinen Zeolith vom Typ ZSM-5. Es handelt sich hierbei um ein kristallines Alumosilicat vom Pentasil-Typ mit einem Verhältnis von Silica zu Alumina von mindestens 12, einem Constraint-Index von 1 bis 12 und einer Porengröße von größer als 0,5 nm.

Die Umsetzung in der zweiten Stufe erfolgt in einem Röhrenreaktor, wobei als niedere Olefine vorzugsweise solche mit drei oder mehr Kohlenstoffatomen (C₃₊-Olefine) erhalten werden. Diese niederen Olefine werden dann an dem ZSM-5-Katalysator unter bestimmten Betriebsbedingungen in Kohlenwasserstoffe im Leichtbenzin-(Gasolin)-Siedebereich umgewandelt. Der Anteil der C₃₊-Olefine und der Gasoline hängt von den Reaktionsbedingungen ab. Die Umsetzung wird jedoch bevorzugt bei erhöhtem Druck durchgeführt, damit das Reaktorvolumen besser ausgenutzt wird.

Ein ähnliches Verfahren ist aus der US 4,471,150 bekannt. Bei diesem Verfahren wird ein mit Magnesiumoxid, Manganoxid oder Magnesiumoxid/Platinoxid modifiziertes kristallines Alumosilicat, z.B. ein Zeolith vom Typ ZSM-34 in der H-Form mit einem verhältnismäßig hohen Alkaligehalt (350 ppm Na, 1,47 % K), verwendet. Die Umsetzung des Methanol- und/oder Dimethyletherdampfes kann in einem Festbett oder in einem Fliessbett mit Verdünnungsmitteln wie Wasserdampf durchgeführt werden, wobei vorzugsweise mehr als 0,5 Mol Wasser je Mol organische Reaktanden eingesetzt werden. Der Gesamtdruck soll vorzugsweise zwischen etwa 0,37 und 3,0 bar liegen, wobei besonders bevorzugt bei Atmosphärendruck gearbeitet wird. Der Propylengehalt des erzeugten Olefingemisches liegt lediglich zwischen 21 und 29 Gew.-%.

Die WO 2009/071654 A1 offenbart die Verwendung eines Katalysators umfassend kristalline Alumosilikate zur Umsetzung von Oxygenaten zu Olefinen. Der verwendete Katalysator wird vor dem Einsatz in der Umsetzungsreaktion von Oxygenaten zu Olefinen einer thermischen Behandlung im alkalischen Zustand unterworfen.

Aus der EP 0 448 000 A1 ist ein Verfahren bekannt, bei dem man zur Erzeugung eines Olefingemisches mit mindestens 5 Gew.-% Ethylen, mindestens 35 Gew.-% Propylen und höchstens 30 Gew.-% Butylen, bezogen auf die Gesamtkohlenwasserstoffe, die Umsetzung unter den folgenden Bedingungen durchführt: (a) bei einem Gesamtdruck von 10 bis 90 kPa, (b) bei einem Gewichtsverhältnis zwischen Wasser und Methanol bzw. Methanoläquivalenten von 0,1 bis 1,5 (c) bei einer Temperatur des Reaktor-Kühlmediums von 280 bis 570 °C, (d) an einem Protonen enthaltenden Katalysator vom Pentasil-Typ, welcher einen Alkaligehalt von weniger als 380 ppm, einen ZnO-Gehalt von weniger als 0,1 Gew.-%, einen CdO-Gehalt von weniger als 0,1 Gew.-%, eine BET-Oberfläche von 300 bis 600 m²/g, eine Primärkristallitgröße von vorzugsweise 0,1 bis 0,9 µm und ein Porenvolumen von 0,3 bis 0,8 cm³/g aufweist.

Aus der EP 1 424 128 A1 ist ein Katalysator auf der Basis von kristallinen Alumosilicaten vom Pentasil-Typ in H-Form bekannt, der aus Primärkristalliten mit einem mittleren Durchmesser von 0,01 und weniger als 0,1 µm aufgebaut ist, welche zu mindestens 20% zu Agglomeraten von 5 bis 500 µm vereinigt sind, wobei der Katalysator peptisierbares Aluminimoxidhydrat als Bindemittel in einer Menge von 10 bis 40 Gew.-% enthält, und wobei der Katalysator eine BET-Oberfläche von 300 bis 600 m²/g und ein Porenvolumen von 0,3 bis 0,8 cm³/g besitzt. Der Katalysator ist für eine Anwendung in einem CMO-Verfahren vorgesehen. Die Selektivität für Propylen im erhaltenen Olefingemisch in den Beispielen liegt bei ca. 35%.

Aus der EP 0 369 364 A2 ist ein Katalysator auf der Basis von kristallinen Alumosilicaten vom Pentasil-Typ in H-Form bekannt, der aus Primärkristalliten mit einem mittleren Durchmesser von mindestens 0,1 und weniger als 0,9 µm aufgebaut ist, welche zu mindestens 20% zu Agglomeraten von 5 bis 500 µm vereinigt sind, wobei der Katalysator feinteiliges Aluminiumoxid als Bindemittel in einer Menge von 10 bis 40 Gew.-% enthält, und wobei der Katalysator eine BET-Oberfläche von 300 bis 600 m²/g und ein Porenvolumen von 0,3 bis 0,8 cm³/g besitzt. Der Katalysator ist für eine Anwendung in einem CMO-Verfahren vorgesehen. Die Selektivität für C₂-C₄-Olefine des Beispiels liegt bei 50 bis 55%.

Es besteht nach wie vor ein Bedarf nach einem Katalysator, der in einem Verfahren zur Herstellung von Olefinen aus Oxygenaten die Erzeugung von Propylen selektiv steigert.

Diese Aufgabe wird erfindungsgemäß dadurch gelöst, dass ein Katalysator, welcher ein SiO₂/Al₂O₃-Gewichtsverhältnis von 2 bis 9 und ein Verhältnis der Peakhöhen der Infrarotabsorptionsbanden v=3610 ±20 cm⁻¹ / v=3680 ±20 cm⁻¹ von 2,1 bis 4,0 aufweist, bereitgestellt wird, wobei der Katalysator einen kristallinen Alumosilicat-Zeolithen und Aluminiumoxid als Bindemittel umfasst und eine BET-Oberfläche von 250 bis 500 m²/g und einen Na-Gehalt unter 200 ppm aufweist.

Die vorliegende Erfindung bezieht sich des Weiteren auf ein Verfahren zur Oligomerisierung von Olefinen.

Die Oligomerisierung von niederen Olefinen, insbesondere C₂-C₆-Olefinen, zur Herstellung von Olefinprodukten mit höherem Molekulargewicht ist ein wirtschaftlich bedeutsames Verfahren beispielsweise im Rahmen der Herstellung von Kraftstoff oder von Vorläufern für Weichmacher und Tenside. Dabei können auch kristalline Alumosilicate als Katalysatoren eingesetzt werden.

Ein derartiges Verfahren ist aus der US 5,672,800 bekannt. Diese Druckschrift beschreibt ein Verfahren zur Oligomerisierung von einem C₂-C₁₂-Alkenenenthaltenden Feed mit einem Wassergehalt von 0,05 bis 0,25 Mol-% über einem Zeolithkatalysator, ausgewählt aus TON, MTT, MFI, MTW, EUO und weiteren Zeolithstrukturen.

Die WO 95/22516 beschreibt ein Olefin-Oligomerisierungsverfahren, umfassend das Inkontaktbringen unter Oligomerisierungsbedingungen eines Olefin-haltigen Feeds mit einem Katalysator umfassend mindestens einen Zeolithen mit einem Constraint-Index von größer 10, wie z.B. ZSM-22, und mindestens einen Zeolithen mit einem Constraint-Index von 2 bis 10, wie z.B. ZSM-5 oder ZSM-57, wobei diese Zeolithe vorzugsweise in einem Gewichtverhältnis von 10:90 bis 90:10 vorliegen. Der Olefinfeed kann zusätzlich ein inertes Verdünnungsmittel wie einen gesättigten Kohlenwasserstoff enthalten.

Es besteht nach wie vor ein Bedarf nach einem Katalysator, der in einem Verfahren zur Oligomerisierung von Olefinen die Ausbeute der Dieselfraktion selektiv steigert.

Diese Aufgabe wird erfindungsgemäß dadurch gelöst, dass ein Katalysator, welcher ein SiO₂/Al₂O₃-Gewichtsverhältnis von 2 bis 9 und ein Verhältnis der Peakhöhen der Infrarotabsorptionsbanden v=3610 ±20 cm⁻¹ / v=3680 ±20 cm⁻¹ von 2,1 bis 4,0 aufweist, bereitgestellt wird, wobei der Katalysator einen kristallinen Alumosilicat-Zeolithen und Aluminiumoxid als Bindemittel umfasst und eine BET-Oberfläche von 250 bis 500 m²/g und einen Na-Gehalt unter 200 ppm aufweist.

Wie vorstehend angegeben liegt der vorliegenden Erfindung die Aufgabe zugrunde, Katalysatoren umfassend Alumosilicat-Zeolithe bereitzustellen, die bei katalytischen Prozessen, insbesondere in einem Verfahren zur Herstellung von niederen Olefinen aus Oxygenaten, vorzugsweise aus Methanol und/oder Dimethylether, oder in einem Verfahren zur Oligomerisierung von Olefinen, eine erhöhte Selektivität aufweisen.

Diese Aufgabe wird durch Katalysatoren gemäß Patentanspruch 1 gelöst, die sich insbesondere durch ein Verhältnis der Peakhöhen der Infrarotabsorptionsbanden v=3610 ±20 cm⁻¹ / v=3680 ±20 cm⁻¹ von 2,1 bis 4,0 auszeichnen.

Die vorliegenden Erfinder fanden überraschend, dass gerade Katalysatoren mit einem bestimmten Verhältnis der Peakhöhen der Infrarotabsorptionsbanden v=3610 ±20 cm⁻¹ / v=3680 ±20 cm⁻¹ von 2,1 bis 4,0 und unter Einhaltung der weiteren in Patentanspruch 1 genannten Merkmale zu verbesserten Selektivitäten und Aktivitäten in bestimmten katalytischen Verfahren führen.

Die vorliegende Erfindung ermöglicht es, maßgeschneiderte Katalysatoren bereitzustellen, die eine selektive Herstellung gewünschter Produkte in bestimmten Reaktionen ermöglichen. Wie bereits beschrieben, war der Einsatz von Katalysatoren auf Alumosilicat-Zeolithbasis in Verfahren zur Herstellung von niederen Olefinen aus Oxygenaten, vorzugsweise aus Methanol und/oder Dimethylether, oder in Verfahren zur Oligomerisierung von Olefinen bereits bekannt. Aus dem Stand der Technik war auch bereits bekannt, dass die Struktur des eingesetzten Zeolithen einen bedeutenden Einfluss auf die Aktivität und Selektivität der katalysierten Reaktion aufweisen kann. Ein Aspekt, der im Stand der Technik nicht hinlänglich untersucht worden war, ist die Frage, welche Eigenschaften ein 'fertiger' Katalysator, umfassend einen kristallinen Alumosilicat-Zeolithen und eine Bindemittelmatrix, aufweisen muss, um in bestimmten Reaktionen eine gewünschte verbesserte Selektivität und Aktivität zu ergeben. Untersuchungen hatten ergeben, dass die Selektivität und Aktivität von reinem Alumosilicat-Zeolith nicht einfach auf jene von Bindemittel-haltigen Katalysatoren auf Alumosilicat-Zeolith extrapoliert werden kann, da ein komplexes Zusammenspiel von physikalischen und chemischen Interaktionen zwischen dem Alumosilicat-Zeolithen und dem Bindemittel stattfindet. In Abhängigkeit der Art, der Menge und der Art der Herstellung der eingesetzten Alumosilicat-Zeolithe und Bindemittel variieren die Stärke an sauren Zentren, deren Verteilung sowie die Konzentration an Aluminium innerhalb des Gerüsts (framework).

Die vorliegenden Erfinder fanden überraschend, dass es trotz der vorstehend beschriebenen komplexen Zusammenhänge möglich ist, anhand eines Parameters, nämlich des Verhältnisses der Peakhöhen der Infrarotabsorptionsbanden v=3610 ±20 cm⁻¹ / v=3680 ±20 cm⁻¹, einen Katalysator zu charakterisieren, der über besonders gute katalytische Eigenschaften verfügt. Es wurde nämlich gefunden, dass sich Katalysatoren mit einem Verhältnis der Peakhöhen der Infrarotabsorptionsbanden v=3610 ±20 cm⁻¹ / v=3680 ±20 cm⁻¹ von 2,1 bis 4,0 durch eine verbesserte Selektivität in einem Verfahren zur Herstellung von niederen Olefinen aus Oxygenaten sowie in einem Verfahren zur Oligomerisierung von Olefinen auszeichnen. Die Infrarotspektroskopie ermöglicht es im Rahmen der vorliegenden Erfindung, über das Verhältnis der Peakhöhen von definierten IR-Absorptionsbanden die besonderen Eigenschaften der verschiedenen sauren OH-Gruppen im Zeolith, am Bindemittel und in der Bindemittel-Zeolith-Grenzfläche zu erfassen. Das Verhältnis der Peakhöhen der Infrarotabsorptionsbanden v=3610 ±20 cm⁻¹ / v=3680 ±20 cm⁻¹ ist 2,1 bis 4,0 bevorzugt 2,1 bis 3,0.

Der erfindungsgemäße Katalysator weist ein SiO₂/Al₂O₃-Gewichtsverhältnis von 2 bis 9, vorzugsweise 2 bis 7 und besonders bevorzugt von 3 bis 6 auf. Darunter ist zu verstehen, dass der Katalysator eine elementare Zusammensetzung aus SiO₂ und Al₂O₃ in einem Gewichtsverhältnis von 2:1 bis 9:1 aufweist.

Der erfindungsgemäße Katalysator umfasst einen kristallinen Alumosilicat-Zeolithen. Geeignete Zeolithmaterialien umfassen Zeolithe mit TON-Struktur (z.B. ZSM-22, ISI-1, KZ-2), MTT-Struktur (z.B. ZSM-23, KZ-1), MFI-Struktur (z.B. ZSM-5), MEL-Struktur (z.B. ZSM-11), MTW-Struktur (z.B. ZSM-12), Zeolithe mit EUO-Struktur oder auch ZSM-21, ZSM-35, ZSM-38, ZSM-4, ZSM-18 oder ZSM-57. Es können auch Mischungen von Zeolithen unterschiedlicher Struktur eingesetzt werden. Vorzugsweise handelt es sich um einen Zeolithen vom Pentasil-Typ, besonders bevorzugt weist der Zeolith eine MFI-Struktur, insbesondere vom ZSM-5 Typ, auf. Vorzugsweise liegen die Zeolithe in der H-Form, d.h. der protonierten Form vor.

Der erfindungsgemäße Katalysator mit einem SiO₂/Al₂O₃-Gewichtsverhältnis von 2 bis 9 enthält neben der Zeolithkomponente noch eine Bindemittelkomponente. Die Bindemittelkomponente liegt im Katalysator in Form einer Matrix vor, in der die kristallinen Alumosilicat-Zeolithe eingebettet sind. Als Bindemittel wird Aluminiumoxid in der Form von peptisierbarem Aluminiumoxidhydrat verwendet. Modifiziertes Aluminiumoxid wie beispielsweise phosphormodifiziertes Aluminiumoxid kann ebenfalls eingesetzt werden. Besonders bevorzugt ist die Verwendung von feinteiligem Aluminiumoxid-Bindemittel, das in der Form von peptisierbarem Aluminiumoxidhydrat eingesetzt wird. Weiter bevorzugt sind mindestens 95 % der Teilchen des peptisierbaren Aluminiumoxidhydrats (auf den mittleren Durchmesser bezogen) ≤ 100 µm.

Des Weiteren ist es bevorzugt, das Bindemittel in einer Menge von 5 bis 60 Gew.-%, noch bevorzugter 10 bis 40 Gew.-%, besonders bevorzugt 15 bis 35 Gew.-%, bezogen auf das Gesamtgewicht des Katalysators (d.h. Alumosilicat und Bindemittel), zu verwenden.

Die BET-Oberfläche des erfindungsgemäßen Katalysators beträgt 250 bis 500 m²/g, vorzugsweise 300 bis 450 m²/g und besonders bevorzugt 320 bis 400 m²/g, bestimmt nach DIN 66131.

Der erfindungsgemäße Katalysator zeichnet sich ferner durch einen niedrigen Na-Gehalt von weniger als 200 ppm, vorzugsweise weniger als 150 ppm aus. Bevorzugt liegt der Na-Gehalt bei weniger als 120 ppm, ganz besonders bevorzugt bei 20 bis 110 ppm, noch bevorzugter zwischen 40 und 80 ppm.

Vorzugsweise beträgt das Porenvolumen des erfindungsgemäßen Katalysators, bestimmt nach der Quecksilberporosimetrie-Methode gemäß DIN 66133, 0,3 bis 0,8 cm³/g.

Die Herstellung eines erfindungsgemäßen Katalysators kann nach dem folgenden Verfahren vorgenommen werden, umfassend die folgenden Schritte:
(a) Kristallisation eines Alumosilicats aus einer wässrigen Suspension, enthaltend eine Siliciumquelle, eine Aluminiumquelle, eine Alkaliquelle und ein Templat,
(b) Abtrennung des Alumosilicats aus der Suspension, gefolgt von einem Trocknungsschritt und einem Calcinierungsschritt zur Entfernung des Templats aus dem Alumosilicat;
(c) Austausch der im Alumosilicat enthaltenden Alkaliionen in wässrigem Medium mit einer protonenhaltigen oder beim Erhitzen Protonen liefernden Substanz, wobei das Molverhältnis der Protonen der protonenhaltigen oder Protonen liefernden Substanz zu Aluminium im Alumosilicat von 5:1 bis 50:1 beträgt, gefolgt von einer optionalen Waschung mit Wasser, bis die Leitfähigkeit des Mediums unter 200 µS/cm liegt, einer Trocknung und einer optionalen Zwischencalcinierung;
(d) Vermischen des ionenausgetauschten Alumosilicats aus Stufe (c) mit einem Bindemittel, wobei das Bindemittel vorpeptisiertes Aluminiumoxidhydrat ist;
(e) Formgebung des Produkts aus Stufe (d); und
(f) Abschlusscalcinierung des Produkts aus Stufe (e).

Bei der Herstellung des erfindungsgemäßen Katalysators ist es zur Erzielung der besonders vorteilhaften katalytischen Eigenschaft von besonderer Bedeutung, dass zur Überführung von der Alkali-Form, insbesondere der Na-Form, in die H-Form mit einer protonenhaltigen oder protonenliefernden Säure das Molverhältnis der Protonen pro vorhandenem Aluminiumatom in einem Bereich von 5:1 bis 50:1 liegt, bevor der Zeolith mit einem Bindemittel vermischt wird und die erhaltene Mischung einer Formgebung unterzogen wird. Der erfinderische Aspekt liegt hier darin, dass die Wechselwirkung zwischen dem unter speziellen Bedingungen während des Ionenaustauschs behandelten Zeolithen mit einem Bindemittel zu einer besonderen Stabilisierung der Aluminiumatome des Zeolithgerüsts führt, die zu einem Verhältnis der Peakhöhen der Infrarotabsorptionsbanden v=3610 ±20 cm⁻¹ / v=3680 ±20 cm⁻¹ von 2,1 bis 4,0 führt.

Am Ende des Verfahrens wird das erhaltene Produkt einer Abschlusskalzinierung unterzogen, wobei hier überraschend gefunden wurde, dass die auf diesem Wege hergestellte Zeolith-Bindemittel-Mischung, d.h. der Katalysator, ganz besonders vorteilhafte katalytische Eigenschaften aufweist, wenn die Abschlusscalcinierung bei relativ geringen Temperaturen im Bereich von 470 bis 650°C für bis 5 Stunden erfolgt.

Die Herstellung der Alumosilicat-Komponente erfolgt vorzugsweise nach dem folgenden Verfahren:
(a) in einem wässrigen Reaktionsansatz, enthaltend eine Siliciumquelle, eine Aluminiumquelle, eine Alkaliquelle und ein Templat, wird bei erhöhter Temperatur und gegebenenfalls bei erhöhtem Druck in an sich bekannter Weise ein alkalisches Alumosilicatgel erzeugt und in ein kristallines Alumosilicat umgewandelt, wobei aber die Reaktion abgebrochen wird, wenn die erhaltenen Primärkristallite einen mittleren Durchmesser von mindestens 0,01 µm, jedoch weniger als 0,1 µm, vorzugsweise von 0,01 bis 0,06 µm, insbesondere von 0,015 bis 0,05 µm haben;
(b) die Primärkristallite werden aus dem wässrigen Reaktionsmedium als Voragglomerate abgetrennt, getrocknet und einer Zwischencalcinierung unterzogen;
(c) das Produkt von Stufe (b) wird zum Austausch der Alkaliionen in wässrigem Medium mit einer protonenhaltigen oder beim Erhitzen Protonen liefernden Substanz umgesetzt, wobei das Molverhältnis der Protonen der protonenhaltigen oder Protonen liefernden Substanz zu Aluminium im Alumosilicat von 5:1 bis 50:1, vorzugsweise 10:1 bis 45:1, besonders bevorzugt von 15:1 bis 40:1, ganz besonders bevorzugt von 20:1 bis 36:1 beträgt, anschließend wird das erhaltene Produkt abgetrennt, gegebenenfalls mit Wasser gewaschen, bis die Leitfähigkeit des Mediums unter 200 µS/cm, vorzugsweise unter 100 µS/cm liegt, getrocknet, und gegebenenfalls erneut einer Zwischencalcinierung unterzogen, worauf eine Agglomeratfraktion von etwa 5 bis 500 µm abgetrennt wird;
(d) die Agglomeratfraktion von Stufe (c) wird mit dem feinteiligen vorpeptisierten Aluminiumoxidhydrat vermischt;
(e) das Produkt aus Stufe (d) wird einer Formgebung unterzogen; und
(f) das Produkt von Stufe (e) wird einer Abschlusscalcinierung unterzogen.

Die Bedeutung der einzelnen Stufen, nach denen der erfindungsgemässe Katalysator erhältlich ist, ist nachstehend näher erläutert:
In der Stufe (a) wird zunächst ein wässriger Reaktionsansatz, enthaltend eine Siliciumquelle (beispielsweise kolloidale Kieselsäure oder ein Alkalisilicat), eine Alkali- und eine Aluminiumquelle (Alkalialuminat, insbesondere Natriumaluminat) und ein Templat, hergestellt. Im Rahmen der vorliegenden Erfindung kann eine Substanz mehrere Funktionen wahrnehmen; beispielsweise kann eine Substanz als Alkaliquelle und als Aluminiumquelle, oder als Siliciumquelle und als Alkaliquelle, oder auch als Alkaliquelle, Aluminiumquelle und Siliciumquelle, dienen. Es wurde gefunden, dass sich bei Verwendung eines Alkalialuminats, insbesondere von Natriumaluminat, als Alkali- und Aluminiumquelle besonders vorteilhafte Katalysatoren erzeugen lassen. In der Stufe a) (Primärsynthese des kristallinen Alumosilicats) erfolgt keine (gesonderte) Säurezugabe. Insbesondere werden im Vergleich zu anderen bekannten Verfahren keine Mineralsäuren wie Schwefelsäure im Reaktionsansatz bei der Primärsynthese verwendet. Dabei werden sowohl die beim Umgang mit (starken) Säuren entstehenden Probleme vermieden, als auch vorteilhafte Katalysatoren erhalten.

Falls der Katalysator nach einer besonders bevorzugten erfindungsgemässen Ausführungsform in einem Verfahren zur Umwandlung von Methanol in Propylen verwendet werden soll, insbesondere einem Verfahren gemäss der DE 100 27 159 A1, deren diesbezügliche Offenbarung hiermit in die Beschreibung aufgenommen wird, werden die Gewichtsanteile zwischen Siliciumquelle und Aluminiumquelle insbesondere so gewählt, dass kristalline Alumosilicate mit einem Si/Al-Atomverhältnis zwischen etwa 50 und 250, vorzugsweise etwa 50 und 150, insbesondere etwa 75 bis 120, noch bevorzugter 85 bis 110 erhalten werden. Falls der fertige Katalysator nach einer weiteren besonders bevorzugten erfindungsgemässen Ausführungsform zur Verwendung in einem Verfahren zur Oligomerisierung vorgesehen ist, werden die Gewichtsanteile zwischen Siliciumquelle und Aluminiumquelle insbesondere so gewählt, dass kristalline Alumosilicate mit einem Si/Al-Atomverhältnis zwischen etwa 10 und 100, vorzugsweise zwischen etwa 20 und 65, insbesondere etwa 25 bis 55, noch bevorzugter 30 bis 50, erhalten werden.

Aus dem Reaktionsansatz wird bei erhöhter Temperatur und gegebenenfalls bei erhöhtem Druck in an sich bekannter Weise ein alkalisches Alumosilicatgel erzeugt. Man kann schon bei Temperaturen ab 90 °C arbeiten, doch werden in diesem Fall die Reaktionszeiten verhältnismäßig lang (etwa 1 Woche). Es wird deshalb vorzugsweise bei Temperaturen von 90 bis 190 °C, insbesondere von 90 bis 150 °C gearbeitet, wobei sich bei Temperaturen von mehr als 100 °C (unter Normalbedingungen) in Abhängigkeit von der Temperatur automatisch ein Überdruck einstellt. Das Alumosilicatgel wandelt sich im Laufe der Reaktion in ein kristallines Alumosilicat um. Wenn die Temperatur des Reaktionsansatzes höher als 190 °C liegt, wird das Wachstum der Alumosilicat-Primärkristallite zu schnell und es werden leicht zu große Primärkristallite erhalten, während gleichzeitig noch Alumosilicatgel im Reaktionsansatz vorhanden ist.

Als Template werden Tetraalkylammoniumverbindungen, vorzugsweise Tetrapropylammoniumhydroxid (TPAOH) oder Tetrapropylammoniumbromid (TPABr) eingesetzt. Man kann als Template auch Gemische aus Ammoniak oder einem organischen Amin und einer weiteren organischen Verbindung aus der Gruppe der Alkohole, vorzugsweise Butanol, verwenden. Der wässrige Reaktionsansatz von Stufe (a) hat vorzugsweise einen pH-Wert von 10 bis 13. Bei einem pH-Wert von weniger als 10 verläuft die Umwandlung des Alumosilicatgels in das kristalline Alumosilicat verhältnismäßig langsam. Bei höheren pH-Werten als 13 können sich die Alumosilicatkristalle in einigen Fällen wieder auflösen. Die Bildung der kristallinen Alumosilicat-Primärkristallite kann durch geeignete Auswahl der Siliciumquelle, der Aluminiumquelle, der Alkaliquelle und des Templats sowie durch geeignete Auswahl der Temperatur und des pH-Werts und der Rührgeschwindigkeit geregelt werden. Bei der Durchführung der Synthese kann die Reaktion abgebrochen werden, wenn die erhaltenen Primärkristallite einen mittleren Durchmesser von mindestens 0,01 µm und weniger als 0,1 µm haben, vorzugsweise im Bereich von 0,01 bis 0,06 µm, insbesondere von 0,015 bis 0,05 µm haben. Zu diesem Zweck werden mehrere Testansätze durchgeführt. Schon nach wenigen Versuchen lassen sich die optimalen Parameter ermitteln, aufgrund derer die erforderlichen Größenbereiche der Primärkristallite erreicht werden. Ein Indiz für die Beendigung der Reaktion besteht auch darin, dass der pH-Wert des Reaktionsansatzes sprunghaft ansteigt. Es braucht nicht in jedem Fall ein neuer Reaktionsansatz hergestellt zu werden. Vielmehr kann zur Erzeugung des Alumosilicatgels die Siliciumquelle, die Alkaliquelle, die Aluminiumquelle, das Templat und das Wasser aus den Mutterlaugen vorheriger Synthesen verwendet und durch die für die Synthese des Alumosilicatgels erforderlichen Mengen der genannten Verbindungen ergänzt werden. Die Messung der mittleren Durchmesser der Primarkristallite erfolgt wie in der EP 1 424 128 A1 beschrieben, deren Offenbarung in die vorliegende Anmeldung aufgenommen wird.

Die Bildung der Alumosilicat-Primärkristallite von Stufe (a) erfolgt vorzugsweise bei einem pH-Wert zwischen 10 und 13, wobei der Reaktionsansatz gerührt wird. Auf diese Weise wird die Größenverteilung der Primärkristallite homogenisiert. Die Rührgeschwindigkeit soll aber vorzugsweise nicht mehr als 900 U/min betragen. Bei größeren Rührgeschwindigkeiten ist der Anteil der kleineren Primärkristallite höher, was gegebenenfalls vorteilhaft ist, solange gewährleistet ist, dass der mittlere Durchmesser aller Primärkristallite mindestens 0,01 µm beträgt.

In der Stufe (b) werden die Primärkristallite aus dem wässrigen Reaktionsmedium als Voragglomerate abgetrennt, d.h. nicht als Einzelkristallite. Dies wird vorzugsweise dadurch erreicht, dass man dem wässrigen Reaktionsmedium ein Flockungsmittel zusetzt. Im allgemeinen wird als Flockungsmittel eine kationische organische makromolekulare Verbindung verwendet. Das Flockungsmittel erleichtert nicht nur die Abtrennung der Primärkristallite aus dem Reaktionsmedium (verbesserte Filtrierbarkeit), sondern bewirkt auch, dass sich die Primärkristallite zu Voragglomeraten zusammenschließen, die hinsichtlich Größe, Struktur und Anlagerung der Primärkristallite schon weitgehend den in der folgenden Stufe gebildeten Agglomeraten gleichen. Die Voragglomerate werden getrocknet und einer Zwischencalcinierung unterzogen, die zunächst vorzugsweise in einer inerten Atmosphäre bei etwa 200 bis 350 °C, insbesondere bei etwa 250 °C durchgeführt wird, wobei ein Teil des Templats bzw. dessen Zersetzungsprodukt desorbiert wird. Die Zwischencalcinierung kann dann in einer oxidierenden Atmosphäre bei etwa 500 bis 600 °C vervollständigt werden, wobei die gegebenenfalls noch vorhandene Restmenge an Templat abgebrannt wird. Im Allgemeinen werden die Voragglomerate etwa 1 bis 20 Stunden in der inerten Atmosphäre und etwa 1 bis 30 Stunden in der oxidierenden Atmosphäre zwischencalciniert.

In der Stufe (c) wird das Produkt von Stufe (b) zum Austausch der Alkaliionen in wässrigem Medium mit einer protonenhaltigen oder beim Erhitzen Protonen liefernden Substanz umgesetzt. Beispielsweise kann der lonenaustausch mit Hilfe einer verdünnten Mineralsäure (z.B. Salzsäure oder Schwefelsäure) oder einer organischen Säure (z.B. Essigsäure) durchgeführt werden. Das Molverhältnis der Protonen der protonenhaltigen oder Protonen liefernden Substanz zu Aluminium liegt im Bereich von 5:1 bis 50:1, vorzugsweise 10:1 bis 45:1, besonders bevorzugt von 15:1 bis 40:1, ganz besonders bevorzugt 20:1 bis 36:1. Dadurch wird ein Verhältnis der Peakhöhen der Infrarotabsorptionsbanden v=3610 ±20 cm⁻¹ / v=3680 ±20 cm⁻¹ von größer 1,8 erhalten. Der lonenaustausch erfolgt vorzugsweise unter Rühren mindestens eine Stunde bei Temperaturen zwischen 25 und 100 °C, wobei zumindest ein Teil der Alkaliionen in den Voragglomeraten der Primärkristallite durch Wasserstoffionen ausgetauscht werden. Falls erforderlich, kann der lonenaustausch unter den gleichen Bedingungen wiederholt werden. Nach dem Austausch der Alkaliionen im wässrigen Medium wird das Protonen enthaltende Produkt (H-Zeolith) abgetrennt (beispielsweise durch Filtration), getrocknet und gegebenenfalls erneut einer Zwischencalcinierung unterzogen. Bevorzugt wird das ionenausgetauschte Produkt nach der Abtrennung (z.B. Filtration oder Sedimentation) und vor der Trocknung solange mit Wasser (destilliertem Wasser) gewaschen, bis die Leitfähigkeit des Mediums (Waschwasser nach Gebrauch) unter 200, vorzugsweise unter 100 µS/cm liegt. Es wurde nämlich überraschend gefunden, dass unter solchen Waschbedingungen erfindungsgemäße Katalysatoren resultieren, die einen sehr niedrigen Na-Gehalt von weniger als 200 ppm, vorzugsweise weniger als 150 ppm, noch bevorzugter weniger als 120 ppm, ganz besonders bevorzugt bei 20 bis 110 ppm, noch bevorzugter zwischen 40 und 80 ppm aufweisen, und die sich durch ein Verhältnis der Peakhöhen der Infrarotabsorptionsbanden v=3610 ±20 cm⁻¹ / v=3680 ±20 cm⁻¹ von größer 1,8 auszeichnen.

Die Zwischencalcinierung wird bei Temperaturen von 400 bis 800 °C, vorzugsweise bei Temperaturen von 450 bis 600 °C über einen Zeitraum von 2 bis 20 Stunden durchgeführt.

Das nach der Zwischencalcinierung erhaltene Produkt enthält einerseits Agglomerate, die ≥ 500 µm sind, und andererseits Staubanteile, die ≤ 5 µm sind. Es wird daher eine Agglomeratfraktion von etwa 5 bis 500 µm abgetrennt.

Diese Agglomeratfraktion wird in der Stufe (d) mit dem feinteiligen Aluminiumoxidhydrat vermischt. Das Aluminiumoxidhydrat zeichnet sich hierbei bevorzugt durch Partikel aus, die zu 95 oder mehr einen Durchmesser von kleiner 100 µm aufweisen.
Diese Angabe ist, gemittelt über eine Vielzahl von Kristalliten, auf den mittleren Durchmesser bezogen, der wie der mittlere Durchmesser der Primärkristallite definiert ist.

Das Aluminiumoxidhydrat ist im Wesentlichen für die Einstellung des Porenvolumens des Katalysators verantwortlich. Die Menge des feinteiligen Aluminiumoxidhydrat-Bindemittels beträgt vorzugsweise etwa 10 bis 40 Gew.-%, bezogen auf das Gesamtgewicht des Produktes (der Mischung) von Stufe (d). Vorzugsweise ist das feinteilige Aluminiumoxidhydrat-Bindemittel peptisierbares Aluminiumoxid, das besonders Na- und Fe-arm ist. Bevorzugt wird zur Peptisierung des Aluminiumoxidhydrats eine Säurekonzentration von 0,15 bis 2,5 mol H⁺/mol Al₂O₃ verwendet, vorzugsweise von 0,20 bis 1,5 mol H⁺/mol Al₂O₃ und insbesondere von 0,4 bis 1,0 mol H⁺/mol Al₂O₃. Die Peptisierung kann prinzipiell mit organischen oder anorganischen Säuren in einem Konzentrationsbereich der Säure von 0,1% bis 100% durchgeführt werden. Beispielsweise können organische Säuren wie 100%ige Essigsäure oder verdünnte anorganische Säuren wie 52%ige Salpetersäure etc verwendet werden.

In der Stufe (e) wird das Produkt von Stufe (d) einer Formgebung unterzogen. Das Verhältnis der Peakhöhen der Infrarotabsorptionsbanden v=3610 ±20 cm⁻¹ / v=3680 ±20 cm⁻¹ ist in dem erhaltenem Produkt 2,1 bis 4,0. Unter Formgebung wird üblicherweise die Überführung eines Materials in Formkörper genau definierter Dimensionen verstanden. Hierzu zählen u.a. Extrudate, Kugel- oder Wabenkörper, Pellets, Granulate und andere Formkörper. Insbesondere kann die Formgebung von einer plastizifierbaren Masse ausgehen, die nach erfolgter Formgebung einer Abschlusscalcinierung (siehe Stufe f) unterzogen wird, um die gewünschte Stabilität zu erhalten.

Das Produkt von Stufe (e) wird einer Abschlusscalcinierung unterzogen. Diese kann allgemein bei Temperaturen zwischen etwa 460 und 850 °C für 1 bis 12 Stunden durchgeführt werden. Vorteilhaft kann die Abschlusscalcinierung bei einer Temperatur von 470 bis 650 °C für bis 5 Stunden, insbesondere von 480 bis 600 °C für 1 bis 5 Stunden, vorzugsweise 1 bis 4 Stunden durchgeführt werden.

Weiterhin bevorzugt kann ein erfindungsgemäßer Katalysator in Ahnlehnung an das vorstehend beschriebene Verfahren hergestellt werden, wobei in Stufe (a) die Reaktion abgebrochen wird, wenn die erhaltenen Primärkristallite einen mittleren Durchmesser von mindestens 0,1 µm und weniger als 0,9 µm haben.

Das so erhaltene Endprodukt kann, wie vorstehend erwähnt, besonders vorteilhaft in Verfahren zur Erzeugung von Olefinen durch die Umwandlung von Oxygenaten oder in Oligomerisierungsverfahren von Olefinen eingesetzt werden.

Prinzipiell ist jedoch auch die Verwendung in anderen Kohlenstoff-Umwandlungsreaktionen, wie insbesondere Dewaxing-Verfahren, Alkylierungen, der Umwandlung von Paraffin zu aromatischen Verbindungen (CPA) sowie verwandten Reaktionen möglich.

Teil der Erfindung ist daher ein Verfahren zur Herstellung von Olefinen aus Oxygenaten, bevorzugt Methanol, Dimethylether oder Mischungen davon, wobei ein Eduktgas, d.h. das gasförmige Ausgangsmaterial, über den erfindungsgemäßen Katalysator geleitet wird. Unter Oxygenaten versteht man im Rahmen der vorliegenden Erfindung Sauerstoffverbindungen, insbesondere organische Sauerstoffverbindungen wie Alkohole und Ether. Die vorliegende Erfindung betrifft daher vorzugsweise ein Verfahren zur Erzeugung von niederen Olefinen aus Sauerstoffverbindungen (Oxygenates to Olefins), vorzugsweise Alkoholen, besonders bevorzugt Methanol (Methanol to Olefins, MTO) durch Umsetzung zum Beispiel eines Methanol- und/oder Dimethyletherdampf und Wasserdampf enthaltenden Reaktionsgemisches in einem Reaktor an einem indirekt gekühlten erfindungsgemäßen Katalysator. Nach dem erfindungsgemäßen Verfahren wird insbesondere die Ausbeute an Propylen pro Zyklus gesteigert.

Die Umsetzung mit dem erfindungsgemäßen Katalysator erfolgt besonders bevorzugt (a) bei einem Gesamtdruck von 10 bis 150 kPa, bevorzugt bei einem Gesamtdruck von 50 bis 140 kPa, (b) bei einem Gewichtsverhältnis zwischen Wasser und Methanol bzw. Methanoläquivalenten von 0,1 bis 4,0, bevorzugt von 0,5 bis 3, und (c) bei einer Temperatur des Reaktor-Kühlmediums von 280 bis 570 °C, bevorzugt von 400 bis 550 °C. Ein solches bevorzugtes Verfahren ist in der EP 0 448 000 A1 beschrieben, deren diesbezügliche Offenbarung hiermit in die Beschreibung aufgenommen wird. Weitere bevorzugte Verfahren sind in der EP 1289912 B1 und der DE 102006026103 A1 beschrieben, deren diesbezügliche Offenbarung hiermit in die Beschreibung aufgenommen wird.

Teil der Erfindung ist ferner ein Verfahren zur Oligomerisierung von Olefinen, wobei ein Eduktgas über den erfindungsgemäßen Katalysator geleitet wird. Das Eduktgas, d.h. gasförmiges Olefinfeed, kann neben Alkenen auch Alkane enthalten. Vorzugsweise enthält das Olefinfeed Propen, Buten (1-Buten, 2-Buten, Isobuten) oder Mischungen davon, gegebenenfalls auch weitere Alkene wie Octen oder Alkane wie Propan. Das Olefinfeed kann auch Wasserstoff oder Stickstoff enthalten, in einem Bereich von 0,1 bis 50 Gew.-%. Das Stoffverhältnis von Propen zu Buten in den Mischungen, die wie vorstehend beschrieben gegebenenfalls noch weitere Komponenten enthalten können, beträgt bevorzugt 60:40 bis 40:60. Weiterhin ist es bevorzugt, wenn das Olefinfeed geringe Mengen an Wasser enthält, im Bereich von 0,05 bis 0,25 Mol%, bezogen auf den Kohlenwasserstoffanteil im Feed. Nach dem erfindungsgemäßen Verfahren wird insbesondere die Ausbeute der Dieselfraktion pro Zyklus gesteigert. Unter Dieselfraktion wird im Rahmen der vorliegenden Anmeldung ein Gemisch aus langkettigen Olefinen, Aliphaten und cyclischen Verbindungen verstanden.

Die Umsetzung mit dem erfindungsgemäßen Katalysator erfolgt besonders bevorzugt (a) mit einem Olefinfeed von 0,5 bis 2,0 kg/kg/h mit der Zusammensetzung von Propen zu 1-Buten im Gewichtsverhältnis 60:40 bis 40:60 und einer Benzinverdünnung im Siedebereich von 45°C bis 170°C, (b) bei einem Gesamtdruck von 1 bis 100 bar (100-10000 kPa), vorzugsweise 30 bis 75 bar (3000-7500 kPa), besonders bevorzugt 35 bis 60 bar (3500-6000 kPa), und (c) bei einer Temperatur des Reaktors von 180 bis 400°C, vorzugsweise 220 bis 320°C.

Die Verwendung der erfindungsgemäßen Katalysatoren in den vorstehend genannten Verfahren ist ebenfalls Gegenstand der Erfindung. Bezüglich bevorzugter Ausführungsformen wird auf die obigen Ausführungen verwiesen.

### Kurze Beschreibung der Abbildungen

Die Abbildung 1 zeigt die Propenselektivität in Abhängigkeit von der Reaktionslaufzeit im Anwendungsbeispiel 1.

Die Abbildung 2 zeigt den Methanolumsatz in Abhängigkeit von der Reaktionslaufzeit im Anwendungsbeispiel 1.

Die vorliegende Erfindung ist durch die nachstehenden Beispiele erläutert.

### Beschreibung der IR-Messungen der Katalysatoren

Für die Messungen wurden die zu messenden Proben tablettiert und anschließend im Vakuum (ca. 10⁻⁶ mbar) bei 450 °C für 1h aktiviert. Die Messung erfolgte nach Abkühlung in der Probenkammer auf Raumtemperatur. Die Einstrahl-IR-Messungen wurden mit einem *Perkin Elmer 2000* Spektrometer aufgenommen. Die Darstellung der Spektren wurde mittels Computer-Software von einer Auftragung der Signalintensitäten zu einer Auftragung der Absorption umgewandelt; zur quantitativen Bestimmung der Peakhöhen wurde zusätzlich eine Grundlinienkorrektur durchgeführt. Die Spektren wurden im Bereich von 4000 bis 800 cm⁻¹ bei einer Auflösung von 2 cm⁻¹ aufgenommen. Über die Intensität der Peakhöhen der Absorptionsbande lässt sich die Anzahl der in der entsprechenden Probe vorliegenden Spezies kalkulieren, die dieser Bande zugeordnet werden können. Zum Vergleich mehrerer Proben untereinander werden die Intensitäten auf einen inneren Standard normiert. Die Normierung erfolgt dabei in Bezug auf die Gerüstschwingung des Alumosilicat-Zeolithen. Zur Beurteilung der relativen Mengen der einzelnen sauren Gruppen wurden die Peakhöhe der Bande bei 3610 ±20 cm⁻¹ zu der Peakhöhe der Bande bei 3680 ±20 cm⁻¹ in Beziehung gesetzt (Intensitätsverhältnis (3610 ±20 cm⁻¹ / 3680 ±20 cm⁻¹).

### Messmethoden

Die Messung der Leitfähigkeit kann mit einem Standard-Leitfähigkeitsmessgerät vorgenommen werden, beispielsweise einem WTW 340i pH/Conductometer mit Leitfähigkeits-Messzelle TetraCon 325. Die Messung erfolgt standardmäßig bei Raumtemperatur.

### Herstellung des erfindungsgemäßen Katalysators 1

Das Alumosilicat, das zur Herstellung des Katalysators 1 eingesetzt wurde, wurde nach folgender Prozedur erhalten: Die Synthese erfolgte durch Vermengen zweier Teilsuspensionen A und B. Suspension A wurde erhalten, indem 69 kg Tetrapropylammoniumbromidsalz in 675 L dest. H₂O gelöst und anschließend mit 155 kg Kieselsäure versetzt wurden. Lösung B wurde durch das Einbringen und Lösen von 19 kg NaOH sowie anschließend 8,35 kg Natriumaluminat in insgesamt 43 L dest. H₂O hergestellt. Die Lösung wurde anschließend mit Lösung A unter innigem Rühren vermengt, mit 10 L dest. H₂O nachgespült, für ca. 30 min weitergerührt und danach in einem druckdichten Synthesekessel auf eine Reaktionstemperatur von 130 °C erhitzt. Die Reaktionssuspension wurde bei dieser Temperatur für 107 h konstant gerührt, anschließend abgekühlt und der dabei entstandene Feststoff durch Filtration von der Mutterlauge abgetrennt. Der so gewonnene Filterkuchen wurde in dest. H₂O wiederum aufgeschlämmt, um überschüssige Fremdionen abzutrennen. Das zeolithische Material wurde mittels eines kationischen Flockungsmittels sedimentiert, die überschüssige Lösung abgepumpt und dieser Waschvorgang mehrmals wiederholt. Der Filterkuchen wurde danach zur Entfernung des Großteils des anhaftenden Wassers vorgetrocknet und anschließend zur Entfernung des organischen Templats calciniert. Dazu wurde der Filterkuchen zunächst unter Stickstoff mit einer Aufheizrate von 60 °C/h auf eine Temperatur von 350 °C erhitzt und bei dieser Temperatur für 16 h gelagert. Anschließend wurde unter Luftatmosphäre mit einer Aufheizrate von 60 °C/h auf 540 °C hoch geheizt und diese Temperatur für 24 Stunden gehalten.
Zur Überführung der Na-Form in die katalytisch aktive H-Form wurden 75 kg der Na-Form mit 375 L einer wässrigen Salzsäure-Lösung versetzt, wobei die Säurekonzentration so gewählt wurde, dass das atomare Verhältnis H⁺ der Salzsäure-Lösung zu Aluminium im Alumosilicat ca. 7 :1 betrug. Es hat sich hier gezeigt, dass die effektive Säureaktivität gering genug war, um das Herauslösen von Aluminiumatomen aus dem Alumosilicatgerüst zu minimieren, was sich besonders auf die katalytischen Eigenschaften des Materials während der Oligomerisierung von Olefinen positiv äussert. Für den lonenaustausch wurde die Säure auf 80 °C erhitzt und danach das Alumosilicat zugegeben. Die Suspension wurde für eine weitere Stunde gerührt und der fein verteilte Feststoff durch Zugabe eines kationischen Flockungsmittels zur Sedimentation gebracht. Die überstehende Lösung wurde abdekantiert und der oben beschriebene lonenaustausch noch ein weiteres Mal durchgeführt, indem weitere 375 L der oben beschriebenen Salzsäurelösung zugegeben wurden. Nach Beendigung des zweiten lonenaustauschs wurde nach erneuter Zugabe des kationischen Flockungsmittels der Feststoff durch Filtration abgetrennt. Der Filterkuchen wurde anschließend gewaschen, um Fremdionen zu entfernen. Die Waschungen wurden abgebrochen, als das gebrauchte Waschwasser eine Leitfähigkeit von ca. 50 µS/cm aufwies. Der Filterkuchen wurde mit einem handelsüblichen Granulator auf eine Partikelgröße von kleiner 2mm zerkleinert und anschließend in einem Calcinationsofen mit einer Aufheizrate von 60 °C/h auf eine Endtemperatur von 540 °C erhitzt und für 10 Stunden bei dieser Temperatur gelagert.

Für die Formgebung wurde das H-Form-Pulver zunächst mittels einer Labormühle auf eine Korngröße von kleiner 100 µm gemahlen und 400 g für das anschließende Forming bereit gestellt. 200 g Aluminiumoxidhydrat, das als Bindemittel eingesetzt wurde, wurde mit 204 ml dest. H₂O zu einer dünnflüssigen Masse verrührt. Im nächsten Schritt wurden 248 g einer Salpetersäurelösung (31 Gew.-% HNO₃) langsam zugetropft, wodurch eine Peptisierung des Aluminiumoxidhydrats einsetzte. Diese hochviskose Paste wurde mit dem Alumosilicatmaterial in einem Kneter solange geknetet, bis es vollständig plastifiziert vorlag, abschließend wurden noch 34 g Steatitöl eingearbeitet. Die Formgebung erfolgte mit einem handelsüblichen Extruder; es wurden Formkörper mit einem Durchmesser von ca. 3 mm und einer Länge von ca. 6 mm erhalten. Eine finale Calcinierung erfolgte bei 600 °C für 5 Stunden.

Der erhaltene Katalysator weist ein SiO₂/Al₂O₃-Gewichtsverhältnis von 4,0. eine BET-Oberfläche von 404 m²/g, und einen Na-Gehalt von 43 ppm, auf. Das Verhältnis der Peakhöhen der IR-Absorptionsbanden bei 3610 ±20 cm⁻¹ und 3680 ±20 cm⁻¹ ist in Tabelle 1 dargestellt.

### Herstellung des Katalysators 2

Das Alumosilicat, das zur Herstellung des nicht erfindungsgemäßen Katalysators 2 eingesetzt wurde, wurde nach folgender Prozedur erhalten: die Synthese erfolgte durch Vermengen zweier Teilsuspensionen A und B. Suspension A wurde erhalten, indem 960 kg Tetrapropylammoniumbromidsalz sowie 4487 kg Kieselsäure in 15550 kg dest. Wasser eingebracht wurden. Lösung B wurde hergestellt, indem 126 kg Natriumaluminat in 1200 kg Natronlauge (8,3 Gew.-%) gelöst wurden. Die Lösung wurde mit Lösung A unter innigem Rühren vermengt, mit weiteren 190 kg einer 50-%igen Natronlauge versetzt, mit 450 L Wasser nachgespült und in einem druckdichten Synthesekessel auf eine Reaktionstemperatur von 130 °C erhitzt. Die Reaktionssuspension wurde bei dieser Temperatur für 60 h konstant gerührt, anschließend abgekühlt und der dabei entstandene Feststoff durch Filtration von der Mutterlauge abgetrennt und anhaftende Fremdionen mit Waschwasser entfernt. Der Filterkuchen wurde danach zur Entfernung des Großteils des anhaftenden Wassers vorgetrocknet und anschließend zur Entfernung des organischen Templats calciniert. Dazu wurde der Filterkuchen zunächst unter Stickstoff mit einer Aufheizrate von 230 °C/h auf eine Temperatur von 250 °C erhitzt und innerhalb von 12 h vorsichtig auf eine Temperatur von 540 °C weiter erhitzt. Anschließend wurde der Zeolith unter Luftatmosphäre bei dieser Temperatur für weitere 6 Stunden gelagert.
Zur Überführung der Na-Form in die katalytisch aktive H-Form wurden 1100 kg der Na-Form mit 5410 L einer wässrigen Salzsäure-Lösung versetzt, wobei die Säurekonzentration so gewählt wurde, dass das atomare Verhältnis H⁺ der Salzsäure-Lösung zu Aluminium im Alumosilicat ca. 15 :1 betrug. Es hat sich hier gezeigt, dass die effektive Säureaktivität gering genug war, um das Herauslösen von Aluminiumatomen aus dem Alumosilicatgerüst zu minimieren, was sich besonders auf die katalytischen Eigenschaften des Materials während der Umwandlung von Olefinen zu Diesel positiv äußert. Für den lonenaustausch wurde die Säure auf 80 °C erhitzt und danach das Alumosilicat zugegeben. Die Suspension wurde zwei weitere Stunden gerührt und der fein verteilte Feststoff durch Zugabe eines kationischen Flockungsmittels zur Sedimentation gebracht. Die überstehende Lösung wurde abdekantiert und der oben beschriebene lonenaustausch noch ein weiteres Mal durchgeführt, indem weitere 5410 L der oben beschriebenen Salzsäurelösung zugegeben wurden. Nach Beendigung des zweiten Ionenaustauschs wurde nach erneuter Zugabe des kationischen Flockungsmittels der Feststoff durch Filtration abgetrennt. Der Filterkuchen wurde anschließend gewaschen, um Fremdionen zu entfernen. Der Filterkuchen wurde anschließend in einem Calcinationsofen unter Luftatmosphäre innerhalb von 8,5 h auf eine Endtemperatur von 540 °C erhitzt und für 2.5 Stunden bei dieser Temperatur gelagert.

Für die Formgebung wurde das H-Form-Pulver zunächst mittels einer Labormühle auf eine Korngröße von kleiner 150 µm gemahlen und 400 g für das anschließende Forming bereitgestellt. 199 g Aluminiumoxidhydrat, das als Bindemittel eingesetzt wurde, wurde mit 204 ml dest. H₂O zu einer dünnflüssigen Masse verrührt. Im nächsten Schritt wurden 248 g einer Salpetersäurelösung (31 Gew.-% HNO₃) langsam zugetropft, wodurch eine Peptisierung des Aluminiumoxidhydrats einsetzte. Diese hochviskose Paste wurde mit dem Alumosilicatmaterial in einem Kneter solange geknetet, bis es vollständig plastifiziert vorlag, abschließend wurden noch 34 g Steatitöl eingearbeitet. Die Formgebung erfolgte mit einem handelsüblichen Extruder; es wurden Formkörper mit einem Durchmesser von ca. 3 mm und einer Länge von ca. 6 mm erhalten. Eine finale Calcinierung erfolgte bei 600 °C für 5 Stunden.

Der erhaltene Katalysator weist ein SiO₂/Al₂O₃-Gewichtsverhältnis von 4,4, eine BET-Oberfläche von 386 m²/g und einen Na-Gehalt von 62 ppm auf. Das Verhältnis der Peakhöhen der IR-Absorptionsbanden bei 3610 ±20 cm⁻¹ und 3680 ±20 cm⁻¹ ist in Tabelle 1 dargestellt.

### Herstellung des erfindungsgemäßen Katalysators 3

Das Alumosilicat, das zur Herstellung des Katalysators 3 eingesetzt wurde, wurde nach folgender Prozedur erhalten: die Synthese erfolgte durch Vermengen zweier Teilsuspensionen A und B. Suspension A wurde erhalten, indem 95.7 kg Tetrapropylammoniumbromidsalz in 600 L dest. H₂O gelöst und anschließend mit 215.8 kg Kieselsäure versetzt wurden, anschließend wurden 30 L dest. H₂O zum Nachspülen zugegeben. Lösung B wurde durch das Einbringen und Lösen von 33.1 kg NaOH sowie anschließend 2,0 kg Natriumaluminat in insgesamt 50 L dest. H₂O hergestellt. Die Lösung wurde mit Lösung A unter innigem Rühren vermengt und nach Nachspülen mit 22 L in einem druckdichten Synthesekessel auf eine Reaktionstemperatur von 130 °C erhitzt. Die Reaktionssuspension wurde bei dieser Temperatur für 60 h konstant gerührt, anschließend abgekühlt und der dabei entstandene Feststoff durch Dekantieren von der Mutterlauge abgetrennt und zur Entfernung von Fremdionen mit Waschwasser gewaschen. Der Filterkuchen wurde danach zur Entfernung des Großteils des anhaftenden Wassers vorgetrocknet und anschließend zur Entfernung des organischen Templats calciniert. Dazu wurde der Filterkuchen zunächst unter Stickstoff mit einer Aufheizrate von 60 °C/h auf eine Temperatur von 350 °C erhitzt und bei dieser Temperatur für 15 h gelagert. Anschließend wurde mit einer Aufheizrate von 60 °C/h unter Luftatmosphäre auf 540 °C hoch geheizt und diese Temperatur für 24 Stunden gehalten.
Zur Überführung der Na-Form in die katalytisch aktive H-Form wurden 70,3 kg der Na-Form mit 346 L einer wässrigen Salzsäure-Lösung versetzt, wobei die Säurekonzentration so gewählt wurde, dass das atomare Verhältnis H⁺ der Salzsäure-Lösung zu Aluminium im Alumosilicat ca. 28 :1 betrug. Es hat sich hier gezeigt, dass die effektive Säureaktivität gering genug war, um das Herauslösen von Aluminiumatomen aus dem Alumosilicatgerüst zu minimieren, was sich besonders auf die katalytischen Eigenschaften des Materials während der Umwandlung von Methanol zu Olefinen positiv geäußert hat. Für den lonenaustausch wurde die Säure auf 80 °C erhitzt und danach das Alumosilicat zugegeben. Die Suspension wurde für eine weitere Stunde gerührt und der fein verteilte Feststoff durch Zugabe eines kationischen Flockungsmittels zur Sedimentation gebracht. Die überstehende Lösung wurde abdekantiert und der oben beschriebene lonenaustausch noch ein weiteres Mal durchgeführt, indem weitere 346 L der oben beschriebenen Salzsäurelösung zugegeben wurden. Nach Beendigung des zweiten lonenaustauschs wurde erneut Flockungsmittel zugegeben und durch Abdekantieren die überstehende Lösung entfernt. Der Filterkuchen wurde anschließend mittels Sedimentationswäsche gewaschen, um Fremdionen zu entfernen. Die Waschungen wurden abgebrochen, als das gebrauchte Waschwasser eine Leitfähigkeit von ca. 80 µS/cm aufwies. Nach diesem Schritt wurde die Zeolithsuspension in eine Filterpresse gegeben, um eine Abtrennung der wässrigen Phase zu erreichen. Der feuchte Filterkuchen wurde anschließend getrocknet, mit einem handelsüblichen Granulator auf eine Partikelgröße von kleiner 2mm zerkleinert und anschließend in einem Calcinationsofen unter Luftatmosphäre mit einer Aufheizrate von 60 °C/h auf eine Endtemperatur von 540 °C erhitzt und für 10 Stunden bei dieser Temperatur gelagert.

Für die Formgebung wurde das H-Form-Pulver zunächst mittels einer Labormühle auf eine Korngröße von kleiner 100 µm gemahlen und 3000 g für das anschließende Forming bereit gestellt. 889 g Aluminiumoxidhydrat, das als Bindemittel eingesetzt wurde, wurde mit 900 ml dest. H₂O zu einer dünnflüssigen Masse verrührt. Im nächsten Schritt wurden 1108 g einer Salpetersäurelösung (31 Gew.-% HNO₃) langsam zugetropft, wodurch eine Peptisierung des Aluminiumoxidhydrats einsetzte. Diese hochviskose Paste wurde mit dem Alumosilicatmaterial in einem Kneter solange geknetet, bis es vollständig plastifiziert vorlag, abschließend wurden noch 252 g Steatitöl eingearbeitet. Die Formgebung erfolgte mit einem handelsüblichen Extruder; es wurden Formkörper mit einem Durchmesser von ca. 3 mm und einer Länge von ca. 6 mm erhalten. Eine finale Calcinierung erfolgte bei 600 °C für 5 Stunden.

Der erhaltene Katalysator weist ein SiO₂/Al₂O₃-Gewichtsverhältnis von 4,6, eine BET-Oberfläche von 334 m²/g und einen Na-Gehalt von 30 ppm auf. Das Verhältnis der Peakhöhen der IR-Absorptionsbanden bei 3610 ±20 cm⁻¹ und 3680 ±20 cm⁻¹ ist in Tabelle 1 dargestellt.

### Herstellung des erfindungsgemäßen Katalysators 4

Das Alumosilicat, das zur Herstellung des Katalysators 4 eingesetzt wurde, wurde nach folgender Prozedur erhalten: die Synthese erfolgte durch Vermengen zweier Teilsuspensionen A und B. Suspension A wurde erhalten, indem 95.7 kg Tetrapropylammoniumbromidsalz in 600 L dest. H₂O gelöst und anschließend mit 215.8 kg Kieselsäure versetzt wurden, anschließend wurden 30 L dest. H₂O zum Nachspülen zugegeben. Lösung B wurde durch das Einbringen und Lösen von 33.1 kg NaOH sowie anschließend 2,0 kg Natriumaluminat in insgesamt 50 L dest. H₂O hergestellt. Die Lösung wurde mit Lösung A unter innigem Rühren vermengt und nach Nachspülen mit 22 L in einem druckdichten Synthesekessel auf eine Reaktionstemperatur von 130 °C erhitzt. Die Reaktionssuspension wurde bei dieser Temperatur für 60 h konstant gerührt, anschließend abgekühlt und der dabei entstandene Feststoff durch Dekantieren von der Mutterlauge abgetrennt und zur Entfernung von Fremdionen mit Waschwasser gewaschen. Der Filterkuchen wurde danach zur Entfernung des Großteils des anhaftenden Wassers vorgetrocknet und anschließend zur Entfernung des organischen Templats calciniert. Dazu wurde der Filterkuchen zunächst unter Stickstoff mit einer Aufheizrate von 60 °C/h auf eine Temperatur von 350 °C erhitzt und bei dieser Temperatur für 15 h gelagert. Anschließend wurde mit einer Aufheizrate von 60 °C/h unter Luftatmosphäre auf 540 °C hoch geheizt und diese Temperatur für 24 Stunden gehalten.
Zur Überführung der Na-Form in die katalytisch aktive H-Form wurden 70,3 kg der Na-Form mit 346 L einer wässrigen Salzsäure-Lösung versetzt, wobei die Säurekonzentration so gewählt wurde, dass das atomare Verhältnis H⁺ der Salzsäure-Lösung zu Aluminium im Alumosilicat ca. 28 :1 betrug. Es hat sich hier gezeigt, dass die effektive Säureaktivität gering genug war, um das Herauslösen von Aluminiumatomen aus dem Alumosilicatgerüst zu minimieren, was sich besonders auf die katalytischen Eigenschaften des Materials während der Umwandlung von Methanol zu Olefinen positiv geäußert hat. Für den lonenaustausch wurde die Säure auf 80 °C erhitzt und danach das Alumosilicat zugegeben. Die Suspension wurde für eine weitere Stunde gerührt und der fein verteilte Feststoff durch Zugabe eines kationischen Flockungsmittels zur Sedimentation gebracht. Die überstehende Lösung wurde abdekantiert und der oben beschriebene lonenaustausch noch ein weiteres Mal durchgeführt, indem weitere 346 L der oben beschriebenen Salzsäurelösung zugegeben wurden. Nach Beendigung des zweiten lonenaustauschs wurde erneut Flockungsmittel zugegeben und durch Abdekantieren die überstehende Lösung entfernt. Der Filterkuchen wurde anschließend mittels Sedimentationswäsche gewaschen, um Fremdionen zu entfernen. Nach diesem Schritt wurde die Zeolithsuspension in eine Filterpresse gegeben, um eine Abtrennung der wässrigen Phase zu erreichen. Der feuchte Filterkuchen wurde anschließend getrocknet, mit einem handelsüblichen Granulator auf eine Partikelgröße von kleiner 2mm zerkleinert und anschließend in einem Calcinationsofen unter Luftatmosphäre mit einer Aufheizrate von 60 °C/h auf eine Endtemperatur von 540 °C erhitzt und für 10 Stunden bei dieser Temperatur gelagert.

Für die Formgebung wurde das H-Form-Pulver zunächst mittels einer Labormühle auf eine Korngröße von kleiner 100 µm gemahlen und 750 g für das anschließende Forming bereit gestellt. 220 g Aluminiumoxidhydrat, das als Bindemittel eingesetzt wurde, wurde mit 500 mL einer Salpetersäurelösung (17 Gew.-% HNO₃) verrührt, wodurch eine Peptisierung einsetzte. Diese hochviskose Paste wurde mit dem Alumosilicatmaterial in einem Kneter solange geknetet, bis es vollständig plastifiziert vorlag, abschließend wurden noch 63 g Steatitöl eingearbeitet. Die Formgebung erfolgte mit einem handelsüblichen Extruder; es wurden Formkörper mit einem Durchmesser von ca. 3 mm und einer Länge von ca. 6 mm erhalten. Eine finale Calcinierung erfolgte bei 600 °C für 5 Stunden.

Der erhaltene Katalysator weist ein SiO₂/Al₂O₃-Gewichtsverhältnis von 4,6, eine BET-Oberfläche von 333 m²/g und einen Na-Gehalt von 47 ppm auf. Das Verhältnis der Peakhöhen der IR-Absorptionsbanden bei 3610 ±20 cm⁻¹ und 3680 ±20 cm⁻¹ ist in Tabelle 1 dargestellt.

### Herstellung des Vergleichskatalysators

Zur Herstellung des Vergleichskatalysators wurden zunächst 10,6 kg Natriumaluminat in 1400 kg einer Natronlaugelösung (6,9 Gew.-%) gelöst. Parallel dazu wurde eine Suspension aus 4545 kg Kieselsäure und 1992 kg Tetrapropylammoniumbromid in 11639 kg dest. Wasser hergestellt. Beide Mischungen wurden anschließend unter ständigem Rühren miteinander vereint, mit weiteren 1224 kg einer Natronlaugelösung (48 Gew.-%) versetzt, mit 1190 kg dest. Wasser nachgespült und in einem druckdichten Reaktionskessel auf eine Temperatur von 130 °C erhitzt und für 60 h konstant gerührt. Anschließend wurde die Reaktionssuspension abgekühlt und der dabei entstandene Feststoff durch Filtration von der Mutterlauge abgetrennt und zur Entfernung von Fremdionen mit Waschwasser gewaschen.
Der Filterkuchen wurde danach zur Entfernung des Großteils des anhaftenden Wassers vorgetrocknet und anschließend zur Entfernung des organischen Templats calciniert. Dazu wurde der Filterkuchen zunächst unter Stickstoff mit einer Aufheizrate von 230 °C/h auf eine Temperatur von 250 °C erhitzt und innerhalb von 12 h vorsichtig auf eine Temperatur von 540 °C weiter erhitzt. Anschließend wurde der Zeolith unter Luftatmosphäre bei dieser Temperatur für weitere 6 Stunden gelagert.
Zur Überführung der Na-Form in die katalytisch aktive H-Form wurden 1500 kg der Na-Form mit 6500 L einer wässrigen Salzsäure-Lösung versetzt, wobei die Säurekonzentration so gewählt wurde, dass das atomare Verhältnis H⁺ der Salzsäure-Lösung zu Aluminium im Alumosilicat ca. 66:1 betrug. Für den lonenaustausch wurde die Säure auf 80 °C erhitzt und danach das Alumosilicat zugegeben. Die Suspension wurde zwei weitere Stunden gerührt und der fein verteilte Feststoff durch Zugabe eines kationischen Flockungsmittels zur Sedimentation gebracht. Die überstehende Lösung wurde abdekantiert und der oben beschriebene lonenaustausch noch ein weiteres Mal durchgeführt, indem weitere 5490 L der oben beschriebenen Salzsäurelösung zugegeben wurden. Nach Beendigung des zweiten lonenaustauschs wurde nach erneuter Zugabe des kationischen Flockungsmittels der Feststoff durch Filtration abgetrennt. Der Filterkuchen wurde anschließend gewaschen, um Fremdionen zu entfernen. Der Filterkuchen wurde anschließend in einem Calcinationsofen unter Luftatmosphäre innerhalb von 8,5 h auf eine Endtemperatur von 540 °C erhitzt und für 2,5 Stunden bei dieser Temperatur gelagert.

Für die Formgebung wurde das H-Form-Pulver zunächst mittels einer Labormühle auf eine Korngröße von kleiner 100 µm gemahlen und 3000 g für das anschließende Forming bereit gestellt. 1838 g Aluminiumoxidhydrat, das als Bindemittel eingesetzt wurde, wurde mit 1880 ml dest. H₂O zu einer dünnflüssigen Masse verrührt. Im nächsten Schritt wurden 2290 g einer Salpetersäurelösung (31 Gew.-% HNO₃) langsam zugetropft, wodurch eine Peptisierung des Aluminiumoxidhydrats einsetzte. Diese hochviskose Paste wurde mit dem Alumosilicatmaterial in einem Kneter solange geknetet, bis es vollständig plastifiziert vorlag, abschließend wurden noch 252 g Steatitöl eingearbeitet. Die Formgebung erfolgte mit einem handelsüblichen Extruder; es wurden Formkörper mit einem Durchmesser von ca. 3 mm und einer Länge von ca. 6 mm erhalten. Eine finale Calcinierung erfolgte bei 600 °C für 5 Stunden.

Der erhaltene Katalysator weist ein SiO₂/Al₂O₃-Gewichtsverhältnis von 4,1, eine BET-Oberfläche von 291 m²/g und einen Na-Gehalt von 26 ppm auf. Das Verhältnis der Peakhöhen der IR-Absorptionsbanden bei 3610 ±20 cm⁻¹ und 3680 ±20 cm⁻¹ ist in Tabelle 1 dargestellt.

**Tabelle 1**

| **Verhältnisse der Peakhöhen (Intensität) der IR-Absorptionsbanden** | | | |
|---|---|---|---|
| **Katalysator** | **Peakhöhe 3680 ±20 cm⁻¹** | **Peakhöhe 3610 ±20 cm⁻¹** | **Verhältnis der Peakhöhen 3610 ±20 cm⁻¹/3680 ±20 cm⁻¹** |
| Katalysator 1 | 0.00179 | 0.00394 | 2.20 |
| Katalysator 2 | 0.00140 | 0.00285 | 2.04 |
| Katalysator 3 | 0.00071 | 0.00208 | 2.93 |
| Katalysator 4* | 0,0622 | 0,1815 | 2.92 |
| Vergleichskat. | 0.00090 | 0.00130 | 1.44 |

| | | | |
|---|---|---|---|
| *Messung mit IR-Spektrometer Thermo Nicolet 4700 mit MCT Detektor | | | |

### Anwendungsbeispiel 1

Ein Test zur Umwandlung von Methanol in Olefin kann durchgeführt werden, indem Eduktgas, insbesondere ein Methanol- und/oder Dimethyletherdampf und gegebenenfalls Wasserdampf enthaltendes Reaktionsgemisch, in einem Reaktor über den erfindungsgemäßen Katalysator geleitet wird. In einem Vergleichsversuch kann der vorstehend beschriebene Vergleichskatalysator verwendet werden. Der Test kann bei Temperaturen im Bereich von 400 bis 550 °C, vorzugsweise 430 bis 500 ° C, vorgenommen werden. Der erfindungsgemäße Katalysator soll zu einer erhöhten Propylenausbeute führen.

Der erfindungsgemäße Katalysator 4 ist in einem Verfahren zur Umwandlung von Oxygenaten zu Olefinen eingesetzt worden. Dabei ist als das Oxygenat ein Methanol- und/oder Dimethyletherdampf und ggf. Wasserdampf enthaltendes Reaktionsgemisch eingesetzt worden, das in einem Reaktor über den erfindungsgemäßen Katalysator geleitet wird. Zum Vergleich wurde ein Katalysator verwendet, der ein Peakverhältnis außerhalb des erfindungsgemäß beanspruchten Bereichs aufweist, siehe Tabelle 1.

Die Reaktion des Anwendungsbeispiels 1 wurde folgendermaßen durchgeführt: Der erfindungsgemäße Katalysator 4 und der Vergleichskatalysator wurden in einen senkrechten Festbettreaktor gefüllt und für 48 h mit Wasserdampf behandelt. Danach wurde die eigentliche Reaktion gestartet, wobei ein Reaktionsgemisch, bestehend aus Methanol und Wasserdampf über den Katalysator geleitet wurden. Die Belastung der Katalysatoren mit Methanol betrug 1/h, d.h. über 1 Gramm Katalysator wurde 1 g Methanol pro Stunde geleitet. Die Temperatur am Reaktoreingang betrug 450 °C, der Test wurde für 425 h durchgeführt.

In Tabelle 2 sind die Selektivitäten und Methanol-Umsatzraten des erfindungsgemäßen Katalysators 4 sowie diejenigen des Vergleichskatalysators für verschiedene Laufzeiten (*tos*) angegeben:

**Tabelle 2**

| **Propenselektivität in Abhängigkeit der Laufzeit** | | | | |
|---|---|---|---|---|
| ***tos* [h)** | **Propen-Selektivität [%]** | | **Methanol-Umsatz [%]** | |
| | **Katalysator 4** | **Vergleichskatalysator** | **Katalysator 4** | **Vergleichskatalysator** |
| 91 | 43.0 | 40.0 | 98.6 | 94.0 |
| 139 | 45.6 | 43.2 | 97.8 | 93.1 |
| 186 | 41.8 | 38.9 | 97.2 | 91.5 |
| 305 | 41.9 | 38.6 | 95.7 | 90.5 |
| 352 | 40.3 | 36.0 | 94.9 | 89.5 |
| 424 | 39.5 | 36.3 | 94.5 | 89.0 |

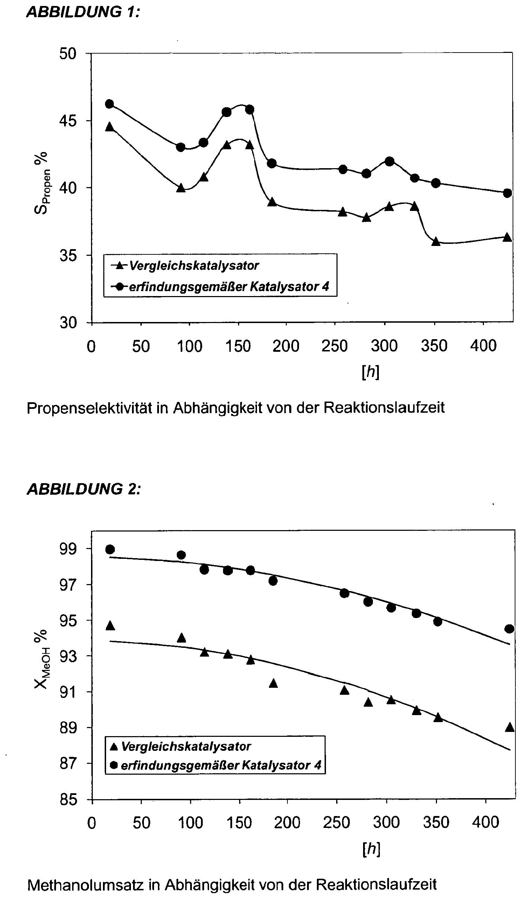

Der Verlauf der Selektivitäten und Methanol-Umsätze ist in Abb. 1 und Abb.2 graphisch dargestellt.

Es zeigt sich eindrucksvoll, dass der erfindungsgemäße Katalysator sowohl im Hinblick auf die Propylenselektivität als auch auf den Methanolumsatz überlegen ist.

### Anwendungsbeispiel 2

Für den Test zur Oligomerisierung von Olefinen kann die Performance des erfindungsgemäßen Katalysators mit der Performance des Vergleichskatalysators verglichen werden. In dem Test kann ein Propen/Buten-Gemisch (optional mit weiteren Komponenten) unter Oligomerisierungsbedingungen umgesetzt werden, wobei der erfindungsgemäße Katalysator zu einer vergleichsweise hohen Ausbeute an Produkten der Dieselfraktion führen soll. Der technische Hintergrund ist, dass es sich zwar um eine Oligomerisierungsreaktion handelt, neben Olefinen aber auch weitere Produkte entstehen, so dass letztlich ein relativ komplexes Gemisch aus langkettigen Olefinen, Aliphaten und cyclischen Verbindungen entsteht, welches als Dieselfraktion bezeichnet wird. Der Nachweis kann durch eine Destillattrennung der Dieselfraktion von weiteren Produktstoffen erfolgen (je mehr Diesel durch destillative Trennung nachgewiesen werden kann, desto besser die Selektivität des Katalysators). Der Test kann insbesondere mit einem Olefinfeed vorgenommen werden, das folgende Komponenten enthält: Propen (vorzugsweise 8 Gew.-%), Propan (vorzugsweise 8 Gew.-%), 1-Buten (vorzugsweise 8 Gew.-%), 1-Octen (vorzugsweise 40 Gew.-%) und Stickstoff (vorzugsweise 36 Gew.-%).

## Patentansprüche

1. Katalysator mit einem SiO₂/Al₂O₃-Gewichtsverhältnis von 2 bis 9, einer BET-Oberfläche von 250 bis 500 m²/g und einem Na-Gehalt unter 200 ppm, wobei der Katalysator einen kristallinen Alumosilicat-Zeolithen und ein Bindemittel umfasst, **dadurch gekennzeichnet, dass** das Verhältnis der Peakhöhen der IR-Absorptionsbanden v=3610 cm⁻¹ ±20 cm⁻¹/ v=3680 cm⁻¹ ±20 cm⁻¹ 2,1 bis 4,0 beträgt und das Bindemittel Aluminiumoxid ist.

2. Katalysator nach Anspruch 1, **dadurch gekennzeichnet, dass** der Alumosilicat-Zeolith eine MFI-Struktur aufweist.

3. Katalysator nach Anspruch 1 oder 2, **dadurch gekennzeichnet, dass** das Verhältnis der Peakhöhen der IR-Absorptionsbanden v=3610 cm⁻¹ ±20 cm⁻¹/ v=3680 cm⁻¹ ±20 cm⁻¹ zwischen 2,1 und 3,0 liegt.

4. Katalysator nach einem der Ansprüche 1 bis 3, **dadurch gekennzeichnet, dass** der Alumosilicat-Zeolith in der H-Form vorliegt.

5. Verfahren zur Herstellung eines Katalysators nach einem der Ansprüche 1 bis 4, umfassend die folgenden Schritte:
(a) Kristallisation eines Alumosilicats aus einer wässrigen Synthesesuspension, enthaltend eine Siliciumquelle, eine Aluminiumquelle, eine Alkaliquelle und ein Templat,
(b) Abtrennung des Alumosilicats aus der Synthesesuspension, gefolgt von einem Trocknungsschritt und einem Calcinierungsschritt zur Entfernung des Templats aus dem Alumosilicat;
(c) Austausch der im Alumosilicat enthaltenden Alkaliionen in wässrigem Medium mit einer protonenhaltigen oder beim Erhitzen Protonen liefernden Substanz, wobei das Molverhältnis der Protonen der protonenhaltigen oder Protonen liefernden Substanz zu Aluminium im Alumosilicat von 5:1 bis 50:1 beträgt, gefolgt von einer Trocknung und einer optionalen Zwischencalcinierung;
(d) Vermischen des ionenausgetauschten Alumosilicats aus Stufe (c) mit einem Bindemittel, wobei das Bindemittel vorpeptisiertes Aluminiumoxidhydrat ist;
(e) Formgebung des Produkts aus Stufe (d); und
(f) Abschlusscalcinierung des Produkts aus Stufe (e).

6. Verfahren gemäß Anspruch 5, wobei im Schritt (c) das erhaltene ionenausgetauschte Produkt abgetrennt wird, gegebenenfalls mit Wasser gewaschen wird, bis die Leitfähigkeit des Mediums unter 200 µS/cm liegt, und anschließend getrocknet und optional zwischencalciniert wird.

7. Verfahren zur Herstellung von Olefinen aus Oxygenaten, bevorzugt aus Methanol, Dimethylether oder Mischungen davon, wobei ein Eduktgas über einen Katalysator gemäß einem der Ansprüche 1 bis 4 geleitet wird.

8. Verwendung eines Katalysators nach einem der Ansprüche 1 bis 4 zur Umwandlung von Oxygenaten zu Olefinen.

9. Verfahren zur Oligomerisierung von Olefinen, wobei ein Eduktgas über einen Katalysator gemäß einem der Ansprüche 1 bis 4 geleitet wird.

10. Verwendung eines Katalysators nach einem der Ansprüche 1 bis 4 zur Oligomerisierung von Olefinen.

## Claims

1. Catalyst having an SiO₂/Al₂O₃ weight ratio of from 2 to 9, a BET surface area of from 250 to 500 m²/g and an Na content below 200 ppm, where the catalyst comprises a crystalline aluminosilicate zeolite and a binder, **characterized in that** the ratio of the peak heights of the IR absorption bands v = 3610 cm⁻¹ ± 20 cm⁻¹/v = 3680 cm⁻¹ ± 20 cm⁻¹ is from 2.1 to 4.0 and the binder is aluminium oxide.

2. Catalyst according to Claim 1, **characterized in that** the aluminosilicate zeolite has an MFI structure.

3. Catalyst according to Claim 1 or 2, **characterized in that** the ratio of the peak heights of the IR absorption bands v = 3610 cm⁻¹ ± 20 cm⁻¹/v = 3680 cm⁻¹ ± 20 cm⁻¹ is between 2.1 and 3.0.

4. Catalyst according to any of Claims 1 to 3, **characterized in that** the aluminosilicate zeolite is present in the H form.

5. Process for producing a catalyst according to any of Claims 1 to 4, comprising the following steps:
(a) crystallization of an aluminosilicate from an aqueous synthesis suspension containing a silicon source, an aluminium source, an alkali metal source and a template,
(b) separation of the aluminosilicate from the synthesis suspension, followed by a drying step and a calcination step to remove the template from the aluminosilicate;
(c) exchange of the alkali metal ions present in the aluminosilicate in aqueous medium by means of a proton-containing substance or a substance which supplies protons upon heating, where the molar ratio of the protons of the proton-containing substance or proton-supplying substance to aluminium in the aluminosilicate is from 5:1 to 50:1, followed by drying and an optional intermediate calcination;
(d) mixing of the ion-exchanged aluminosilicate from step (c) with a binder, where the binder is prepeptised aluminium oxide hydrate;
(e) shaping of the product from step (d); and
(f) final calcination of the product from step (e) .

6. Process according to Claim 5, wherein, in step (c), the ion-exchanged product obtained is separated, optionally washed with water until the conductivity of the medium is below 200 µS/cm and subsequently dried and optionally subjected to intermediate calcination.

7. Process for preparing olefins from oxygenates, preferably from methanol, dimethyl ether or mixtures thereof, wherein a feed gas is passed over a catalyst according to any of Claims 1 to 4.

8. Use of a catalyst according to any of Claims 1 to 4 for converting oxygenates into olefins.

9. Process for oligomerizing olefins, wherein a feed gas is passed over a catalyst according to any of Claims 1 to 4.

10. Use of a catalyst according to any of Claims 1 to 4 for the oligomerization of olefins.

## Revendications

1. Catalyseur présentant un rapport pondéral SiO₂/Al₂O₃ de 2 à 9, une surface BET de 250 à 500 m²/g et une teneur en Na inférieure à 200 ppm, le catalyseur comprenant une zéolithe cristalline de silicate d'aluminium et un liant, **caractérisé en ce que** le rapport des hauteurs de pic des bandes d'absorption des IR (v = 3610 cm⁻¹ ± 20 cm⁻¹) / (v = 3680 cm⁻¹ ± 20 cm⁻¹) = 2,1 à 4,0 et le liant est de l'oxyde d'aluminium.

2. Catalyseur selon la revendication 1, **caractérisé en ce que** la zéolithe de silicate d'aluminium présente une structure MFI.

3. Catalyseur selon la revendication 1 ou 2, **caractérisé en ce que** le rapport des hauteurs de pic des bandes d'absorption des IR (v = 3610 cm⁻¹ ± 20 cm⁻¹) / (v = 3680 cm⁻¹ ± 20 cm⁻¹) est situé entre 2,1 à 3,0.

4. Catalyseur selon l'une quelconque des revendications 1 à 3, **caractérisé en ce que** la zéolithe de silicate d'aluminium se trouve sous la forme H.

5. Procédé pour la préparation d'un catalyseur selon l'une quelconque des revendications 1 à 4, comprenant les étapes suivantes :
(a) cristallisation d'un silicate d'aluminium à partir d'une suspension de synthèse aqueuse, contenant une source de silicium, une source d'aluminium, une source de métal alcalin et une matrice,
(b) séparation du silicate d'aluminium de la suspension de synthèse, suivie d'une étape de séchage et d'une étape de calcination pour l'élimination de la matrice à partir du silicate d'aluminium ;
(c) échange des ions de métal alcalin contenus dans le silicate d'aluminium en milieu aqueux avec une substance contenant des protons ou libérant des protons lors du chauffage, le rapport molaire des protons de la substance contenant ou libérant des protons à l'aluminium dans le silicate d'aluminium étant de 5:1 à 50:1, suivi d'un séchage et d'une calcination intermédiaire optionnelle ;
(d) mélange du silicate d'aluminium à ions échangés de l'étape (c) avec un liant, le liant étant de l'oxyde d'aluminium hydraté prépeptisé ;
(e) façonnage du produit de l'étape (d) ; et
(f) calcination finale du produit de l'étape (e).

6. Procédé selon la revendication 5, le produit à ions échangés obtenu dans l'étape (c) étant séparé, le cas échéant lavé avec de l'eau jusqu'à ce que la conductibilité du milieu se situe sous 200 pS/cm et ensuite séché et éventuellement calciné de manière intermédiaire.

7. Procédé pour la préparation d'oléfines à partir de produits oxygénés, de préférence à partir de méthanol, de diméthyléther ou de leurs mélanges, un gaz de départ étant guidé sur un catalyseur selon l'une quelconque des revendications 1 à 4.

8. Utilisation d'un catalyseur selon l'une quelconque des revendications 1 à 4 pour la conversion de produits oxygénés en oléfines.

9. Procédé pour l'oligomérisation d'oléfines, un gaz de départ étant guidé sur un catalyseur selon l'une quelconque des revendications 1 à 4.

10. Utilisation d'un catalyseur selon l'une quelconque des revendications 1 à 4 pour l'oligomérisation d'oléfines.
